# EUROPÄISCHE PATENTSCHRIFT

(11) **EP 2 854 699 B1**
(45) Veröffentlichungstag und Bekanntmachung des Hinweises auf die Patenterteilung: **30.03.2016**
(21) Anmeldenummer: 13728333.9
(22) Anmeldetag: 27.05.2013
(51) Int. Cl.: A61B 6/14, A61C 19/045, G06T 7/00, G06T 19/20, A61C 9/00

(54) **VERFAHREN ZUM ERSTELLEN EINES VIRTUELLEN KIEFERABBILDES**
METHOD FOR CREATING A VIRTUAL JAW IMAGE
PROCÉDÉ DE PRODUCTION D'UNE IMAGE VIRTUELLE DES MÂCHOIRES

(30) Priorität: 25.05.2012 DE 102012104543; 06.06.2012 DE 102012104912
(43) Veröffentlichungstag der Anmeldung: 08.04.2015
(73) Patentinhaber: Sicat GmbH & CO. KG, 53177 Bonn (DE)
(72) Erfinder: KUSCH, Jochen, 53343 Wachtberg (DE); HANSSEN, Nils, 53111 Bonn (DE)
(74) Vertreter: Braun-Dullaeus Pannen Patent- und Rechtsanwälte
(86) Internationale Anmeldenummer: PCT/EP2013/060870
(87) Internationale Veröffentlichungsnummer: WO 2013/175018

(56) Entgegenhaltungen:
- US-A- 4 836 778
- US-A- 6 152 731
- US-A1- 2003 204 150
- US-A1- 2013 130 195

## Beschreibung

Die Erfindung betrifft ein Verfahren zum Erstellen eines virtuellen Kieferabbildes.

In der funktionellen Zahnheilkunde wird ein Gesamtsystem aus Knochen, Zähnen, Kiefergelenken und Muskeln betrachtet. Komplexe patientenindividuelle Bewegungsabläufe des gesamten Systems werden zunehmend mit elektronischen Verfahren aufgezeichnet, die die Lage des Unterkiefers in Relation zum Oberkiefer zu unterschiedlichen Zeitpunkten in sechs Freiheitsgraden (drei Rotationen, drei Translationen) erfassen können. Solche Bewegungsaufzeichnungen können als Kondylographiedaten vorliegen.

In der DE 10 2004 002 953 A1 wird ein Verfahren beschrieben, in dem die Messung der Relativbewegungen der Kiefer anhand zweier starr mit dem Ober- bzw. dem Unterkiefer verbundener Ultraschallgeber und -sensoren erfolgt. Durch eine aufbereitete Darstellung der so gewonnenen Kondylographiedaten kann der Zahnarzt z.B. auch die Bewegungsbahn einer gedachten Scharnierachse der Kiefergelenke beim Kauvorgang darstellen. Diese aufbereitete Darstellung der Bewegungsbahn einer gedachten Scharnierachse entspricht der Messung, wie sie ältere Messsysteme - sog. Achsiographiesysteme - meist mechanisch direkt aufzeichnen. Solche Achsiographiesysteme messen nicht die vollen sechs Freiheitsgrade der Unterkieferlage, sondern nur eine Winkel-Weg-Kombination, aus der die räumliche Lage des Unterkiefers in Relation zum Oberkiefer meist nicht eindeutig ermittelt werden kann. Trotzdem erfolgt auch bei Kondylographiesystemen mit vollen sechs Freiheitsgraden oft die Darstellung der Achsspur, weil Ärzte entsprechend geschult sind, auf Basis dieser Spurdaten Pathologien zu diagnostizieren.

Auch wenn viele Pathologien in der Gelenk- und Muskelanatomie durch Kondylographien bzw. Achsiographien diagnostiziert werden können, muss die Diagnose häufig durch bildgebende Verfahren differenziert werden, die eine verborgene Anatomie geometrisch korrekt darstellen. Beispiele für solche bildgebenden Verfahren sind die digitale Volumentomographie (DVT), die Magnetresonanztomographie (MRT) und die Computertomographie (CT). Auch für die an die Diagnose anschließende Therapieplanung ist die Anfertigung von tomographischen Bildern oft essentiell. Insbesondere dann, wenn Veränderungen am Knochen - z.B. während einer kieferorthopädischen Behandlung oder in einem chirurgische Eingriff - vorgenommen werden müssen.

Bisher werden die Lagebeziehungen der Kiefer während eines tomographischen Scans typischerweise gar nicht eingestellt. Der Patient wird vielmehr in einer undefinierten Kieferstellung gescannt, indem er mit einem generischen Aufbiss fixiert wird oder sein Kopf auf einer Kinnstütze aufliegt. Wird ein Aufbiss verwendet sind die Zähne leicht geöffnet; im Falle der Kinnstütze sind die Zähne in Okklusion bzw. maximaler Interkuspidation.

Einige Aufnahmeprotokolle, insbesondere in der Magnetresonanztomographie, schreiben eine bestimmte Kieferöffnung vor, um die Lage des Discus im Kiefergelenk diagnostizieren zu können. Diese Kieferöffnung wird typischerweise durch einen Zylinder von einigen Zentimetern Durchmesser hergestellt, den der Patient während der Aufnahme zwischen den Zähnen hält. Die Öffnung des Patientenmundes durch einen Zylinder erlaubt jedoch nur eine sehr grobe Beeinflussung der Relation von Ober- und Unterkiefer zueinander. Außerdem sind damit nur Relationen fixierbar in denen sich die Kiefergelenke in einer zentrischen Position befinden. Quer- und Hebelkräfte lassen sich durch einen Zylinder nicht ausüben.

Erschwerend kommt hinzu, dass Patienten häufig von einem Kiefer- oder Zahnspezialisten an einen Fachmann zur Aufnahme von tomographischen Volumendaten überwiesen werden, der jedoch in der Regel über keine Fachkunde im Dentalbereich verfügt. Solche Fachleute sind in der Regel nicht in der Lage, die genaue Positionierung des Kiefers zu überprüfen oder einzustellen.

Bei der Diagnose und Behandlung von Kiefergelenksproblemen sollte bei jeglichen Aufnahmen der Kiefer grundsätzlich darauf geachtet werden, dass möglichst keine unnatürlichen bzw. unerwünschten Kräfte auf die Kiefer wirken. So kann es beispielsweise bei der Durchführung eines Tomographieverfahrens in der Interkuspidationsposition (IKP) dazu kommen, dass aufgrund der Muskelkraft eine, wenn auch geringe Verformung der Kiefer stattfindet oder die Kiefergelenke in eine unnatürliche Stellung einnehmen. Dies würde natürlich die Qualität der digitalen Abbilder deutlich schmälern. Ferner eigenen sich tomographische wie auch andere Aufnahmen, die in der IKP aufgenommen wurden grundsätzlich eher schlecht, da die Bilddaten in den Grenzbereichen zwischen Ober- und Unterkiefer häufig nur schlecht automatisiert dem jeweiligen Kiefer zugeordnet werden können.

Die US 6,152,731 A offenbart ein Verfahren zum Anzeigen der Okklusion der Zähne bei einem Patienten. Dabei wird eine Gelenksachse ermittelt, anhand derer die Zähne der Oberkiefer und Unterliefer in entsprechende Relativstellungen zueinander gebracht werden können.

Die US 2003/0204150 A1 zeigt ein Verfahren zu Untersuchung von Kieferbewegungen. Dabei werden Oberflächendaten der Zähne erfasst. Ebenfalls ein Verfahren zum Erfassen von Kieferbewegungen ist in der US 4, 836,778 A beschrieben.

Es ist Aufgabe der vorliegenden Erfindung, ein aussagekräftiges Diagnosewerkzeug zum Diagnostizieren von Kiefergelenkserkrankungen zu schaffen.. Die der Erfindung zugrundeliegende Aufgabe wird gelöst durch ein Verfahren nach Anspruch 1; vorteilhafte Ausgestaltungen ergeben sich aus den Unteransprüchen.

Der Kern der Erfindung liegt insbesondere darin, dass zu einem beliebigen Zeitpunkt und damit in einer nicht näher definierten Kieferstellung erstellte digitale Abbilder des Oberkiefers und des Unterkiefers, also z.B. in DVT-Scans o.ä., anhand der Bewegungsaufzeichnung nunmehr in eine beliebig vorgebbare Stellung zueinander gebracht werden können, die zudem einer realen Stellung des Unterkiefers gegenüber dem Oberkiefer entspricht. Dabei kann beispielsweise in einem ersten Diagnoseschritt anhand der Bewegungsaufzeichnung zu einem bestimmten Zeitpunkt auf eine Pathologie geschlossen werden. Einzelheiten zu dieser Pathologie können der Bewegungsaufzeichnung allein allerdings oft nur begrenzt entnommen werden. Anhand der Erfindung wird nun das Kiefergelenk in der zu begutachtenden Stellung von Unterkiefer und Oberkiefer gezeigt werden, woraus sich bereits weitere Befunde und Diagnosen erzeugen lassen, ohne dass ein zusätzliches weiteres Bildgebungsverfahren bei gewünschter Kieferstellung hier zwangsläufig erforderlich wäre. Ebenso können auch andere Zeitpunkte, nämlich insbesondere ein Zeitpunkt kurz vor dem zuvor bestimmten Zeitpunkt oder ein Zeitpunkt kurz nach dem oben bestimmten relevanten Zeitpunkt ausgewählt werden. Die entsprechende Stellung der Kiefer zueinander kann durch einfaches virtuelles Ausrichten des digitalen Unterkieferabbildes gegenüber dem digitalen Oberkieferabbild visualisiert werden. Hierdurch werden folglich Diagnosemöglichkeiten geschaffen, die bisher noch unbekannt sind und allenfalls durch bewegte Tomographieverfahren zu ersetzen gewesen wären, die aber wesentlich aufwendiger sind und den Patienten beträchtlich mehr ionisierender Strahlung aussetzen würden. Der Benutzer kann nun den gewünschten Zeitpunkt der Bewegungsaufzeichnung am Computer einstellen und kann dann eine graphische Repräsentation der Kieferstellung erhalten.

Als Bewegungsaufzeichnungsverfahren wird insbesondere das Kondylographieverfahren verwendet, welches in der nachfolgenden Beschreibung auch stellvertretend für andere Bewegungsaufzeichnungsverfahren genannt wird. Das Ergebnis eines Bewegungsaufzeichnungsverfahrens ist die Bewegungsaufzeichnung, wobei nachfolgend stellvertretend für die Ergebnisse anderer Verfahren auch von dem Kondylogramm oder den Kondylographiedaten gesprochen wird.

Die verwendeten digitalen volumetrischen Abbilder der Kiefer stammen dabei von der Person selbst, an der das Kondylographieverfahren durchgeführt wurde. Vorzugsweise werden dabei das Oberkieferabbild und das Unterkieferabbild während einer beliebigen Kieferstellung jeweils durch ein Bildgebungsverfahren erzeugt. Die Stellung des Unterkiefers gegenüber dem Oberkiefer während des Bildgebungsverfahrens kann daher insbesondere eine solche sein, die von der virtuellen Ausrichtung des zu erzeugenden virtuellen Kieferabbildes abweicht. Insofern ist es also nicht mehr zwangsläufig erforderlich, die digitalen Abbilder des Oberkiefers und des Unterkiefers in exakt einer solchen Stellung aufzunehmen, zu der das Kondylogramm auf eine mögliche Pathologie hingedeutet hat. Es reicht vielmehr aus, dass die realen Kiefer zunächst in "irgendeiner" Stellung aufgenommen werden, um dann anhand der oben angesprochenen Simulation jegliche Kieferstellung zumindest virtuell nachzubilden. Mit anderen Worten wird durch das erfindungsgemäße Verfahren der Fachmann in die Lage versetzt, Kondylographiedaten und volumetrische, insbesondere tomographische Aufnahmen zu fusionieren.

Zum Auswählen des Positionsdatensatzes wird ein Zeitpunkt im Verlauf der Bewegungsaufzeichnung ausgewählt und anhand des ausgewählten Zeitpunktes wird dann der zugehörige Positionsdatensatz identifiziert und damit ausgewählt. Die Positionsdatensätze werden dabei über eine gewisse Zeitdauer aufgenommen, wobei jedem Positionsdatensatz ein Zeitpunkt der Kaubewegung zumindest mittelbar zuzuordnen ist. Der Zeitpunkt muss dabei nicht zwangsläufig in einer zeitlichen Einheit abgebildet sein, sondern kann auch beispielsweise dem Mundöffnungswinkel während des Öffnens und des Schließens zuzuordnen sein.

Der Positionsdatensatz kann dabei eine örtliche Stellung zumindest eines oder mehreren ortsfest mit dem Unterkiefer verbundenen Punkten gegenüber einem Bezugssystem darstellen, welches fest mit dem Oberkiefer verbunden ist, jeweils zum Zeitpunkt der Aufnahme im Kondylographieverfahren. Wie der Unterkiefer selbst dann absolut zum Oberkiefer angeordnet ist, ist den Positionsdatensätzen selbst nicht zwangsläufig unmittelbar zu entnehmen. Die Stellung des Unterkiefers gegenüber dem Oberkiefer ist aber an zumindest einem Zeitpunkt des Kondylogramms entweder bereits bekannt oder kann noch ermittelt werden; hierzu kann insbesondere durch ein Bissregistrat die absolute Ausrichtung des Oberkiefers zum Unterkiefer bezüglich zumindest eines einzelnen Positionsdatensatzes ermittelt werden. Hiermit können dann auch zu den übrigen Positionsdatensätzen die korrespondierenden Kieferlagen ermittelt werden. Die Ausrichtung kann dabei beispielsweise derart erfolgen, dass ein Oberflächenscan des Bissregistrats durchgeführt wird. Die Oberflächendaten der Oberkiefer und Unterkiefer sind ja bereits aus den jeweiligen digitalen Ober- und Unterkieferabbildern bekannt. Dann wird ein "Matching" vorgenommen: Das bedeutet, dass der Positionsdatensatz zum definierten Zeitpunkt aufgerufen wird und dann die Oberflächendaten des Bissregistrats zum digitalen Unterkieferabbild und dann dass Bissregistrat zum digitalen Oberkieferabbild virtuell ausgerichtet wird. Damit wird dann die Ausrichtung zwischen digitalem Oberkieferabbild und digitalem Unterkieferabbild hergestellt. Die weiteren Ausrichtungen der beiden Kieferabbilder zu anderen Zeitpunkten können dann durch "Weiterspulen" des Kondylogramms abgespielt werden.

Zusammengefasst werden in dem erfindungsgemäßen Verfahren folglich z.B. tomographische Daten (digitale Kieferabbilder) des Kiefers und des Unterkiefers beschafft oder erstellt. Die relative Lage dieser beiden Kiefer zueinander ist bei der Erstellung der Daten gleichgültig, da es nur um die Daten der Kiefer an sich geht. Separat dazu wird beispielsweise ein an sich bekanntes Kondylographieverfahren (Bewegungsaufzeichnungsverfahren) durchgeführt, durch welches die Relativbewegungen der Kiefer ermittelt werden. Während des Kondylogprahieverfahrens wird zumindest einmal ein Bissregistrat erstellt oder anderweitig eine relative Lage zwischen Oberkiefer und Unterkiefer festgestellt (Abtasten der Oberflächenabschnitte zum definierten Zeitpunkt). Damit werden dann die tomographischen Daten der beiden Kiefer zueinander räumlich synchronisiert. Man erhält folglich einen Datenwert aus dem Kondylographieverfahren, zu dem die korrekte räumliche Ausrichtung der beiden Kieferabbilder zueinander bekannt ist. Verändert man nun die relative Ausrichtung der beiden Kieferabbilder auf einen entsprechenden anderen Datensatz des Kondylographieverfahrens, so erhält man die relative Ausrichtung der beiden digitalen Kieferabbilder, die genau der realen Ausrichtung der realen Kiefer entspricht, als dieser andere Datensatz an der realen Testperson erzeugt wurde. Dieser andere Datensatz kann nun beispielsweise ein solcher sein, bezüglich dem die Kondylographie auf eine Pathologie hindeutet. Man kann sich nun die digitalen Kieferabbilder in dieser mutmaßlich pathologischen Stellung ansehen und entsprechend auswerten.

Bevorzugt kann während des Abtastens alternativ ein Oberflächenabbild, beispielsweise eine 3D-Photographie, des realen Oberkiefers und des realen Unterkiefers gemacht werden, wobei der Zeitpunkt dieser 3D-Photographie einem Zeitpunkt der Bewegungsaufzeichnung entspricht. Die 3D-Photographie kann dann beispielsweise durch ein Verfahren mit den digitalen Ober- und Unterkieferabbildern verknüpft werden, wie es in der DE 10 2007 001 684 A1 beschrieben ist.

Zwar könnte das Ausrichten bereits dadurch erfolgen, dass das digitale Oberkieferabbild und das Unterkieferabbild bereits in Interkuspidation aufgenommen wurden. Jedoch ist die eindeutige Zuordnung der Ober- und Unterkieferabdrücke anhand der ineinandergreifenden Zahnhöcker nicht immer eindeutig bestimmbar. Weiterhin bietet das erfindungsgemäße Verfahren demgegenüber aber folgende Vorteile: Durch die Möglichkeit, Oberkieferabbilder und Unterkieferabbilder zu verwenden, die in "irgendeiner" Kieferstellung aufgenommen wurden kann sichergestellt werden, dass diese Kieferabbilder keine Verspannungen oder Verformungen aufweisen, die sich durch den Zusammenbiss ergeben könnten.

Unter dem bestimmten Zeitpunkt ist insbesondere auch eine bestimmte Position im Graphen des Achsiogramms oder in den Positionsdatensätzen des Kondylogramms gemeint.

Die Erfindung betrifft ferner ein Verfahren zum Erzeugen einer Scanschiene, wobei ein Verfahren zum Erstellen einer Simulation nach der oben beschriebenen Art durchgeführt wird. Dabei wird ferner anhand des virtuellen Kieferabbildes eine Scanschiene erzeugt, wobei die Scanschiene zumindest eine definierte Oberkieferanlagefläche und eine definierte Unterkieferanlagefläche aufweist. Die Oberkieferanlagefläche und die Unterkieferanlagefläche sind dabei derart zueinander ausgerichtet, dass der reale Unterkiefer zum realen Oberkiefer die räumliche Stellung entsprechend des ausgewählten Positionsdatensatzes einnimmt, wenn die Oberkieferanlagefläche in Anlage mit dem Oberkiefer und die Unterkieferanlagefläche in Anlage mit dem Unterkiefer ist. Unter Oberkiefer und Unterkiefer sind dabei jeweils die realen Ober- und Unterkiefer gemeint. Unter in Anlage mit einem Kiefer zu sein bedeutet im Rahmen der vorliegenden Erfindung aber auch, eine zumindest mittelbare Anlage mit dem Kiefer, wobei die unmittelbare Anlage mit einem ortsfest zum Kiefer gehaltenen Gegenstand, insbesondere einem Zahn zu verstehen ist. Die Anlage mit einem Zahn entspricht dabei auch der Anlage mit dem zugehörigen Kiefer. Die Kieferanlageflächen können dabei insbesondere solche Oberflächen sein, die zumindest abschnittsweise invers zu Zahnoberflächen ausgebildet sind. Die Kieferanlageflächen können dann zu einem gemeinsamen Objekt, nämlich der Scanschiene verbunden werden. Die Scanschiene umfasst dann dabei geometrische Oberflächenabschnitte der beiden Kiefer, die zueinander exakt so ausgerichtet sind, wie die realen Oberflächenbereiche der Kiefer in der ausgewählten Kieferstellung. Eine solche Schiene kann dann beispielsweise durch eine Fräse, ein Stereolithographieverfahren oder einen 3D-Druck gebildet werden. Anhand der Scanschiene lassen sich somit reale Kieferstellungen, die anhand des Kondylogramms als auffällig diagnostiziert wurden, jederzeit real reproduzieren. Anhand der Scanschiene können dann Untersuchungen am realen Kiefer in der Kieferstellung entsprechend des ausgewählten Positionsdatensatzes durchgeführt werden.

In einem weiteren nachfolgenden Verfahrensschritt können dann auch digitale Volumendaten erzeugt werden, wobei die Scanschiene in Anlage mit dem Unterkiefer und dem Oberkiefer gebracht wird und anschließend der Oberkiefer und der Unterkiefer mit jeweils angelegter Scanschiene einem tomographischen Bildaufnahmeverfahren zugeführt werden. Insbesondere durch ein tomographisches Bildaufnahmeverfahren kann dann ein neues Abbild erzeugt werden, wobei der während Bildaufnahme der reale Unterkiefer in der Stellung entsprechend des ausgewählten Positionsdatensatzes zum realen Oberkiefer gehalten ist. Der Patient trägt während des Aufnahmeverfahrens die Scanschiene, so dass sichergestellt ist, dass während des Aufnahmeverfahrens die Kiefer in der gewünschten Stellung sind. In den vorherigen Verfahrensschritten wurde also zunächst festgestellt, welche Kieferlage problematisch und einer weiteren Untersuchung bedarf und wie es möglich ist diese reale Kieferlage wiederherzustellen. Dann wurde die Scanschiene hergestellt, mit deren Hilfe der realen Kiefer nun stets in die problematische Lage gebracht werden kann, nämlich insbesondere zum Zweck, um konkret in der problematischen Lage einen Scan, insbesondere eine DVT durchzuführen. Der essentielle Vorteil liegt nun darin, dass die Scanschiene die Kiefer in eine natürlich Stellung zueinander bringt, da diese Stellung einem Datensatz des Kondylographiverfahrens entspricht.

Gegenüber dem bereits oben dargestellten virtuellen Kieferabbild können durch das weitere Abbild nun auch oder nur nichtstarre Komponenten des Kiefers oder angrenzender Gewebebereiche, insbesondere flexible Teile wie Knorpel, Muskeln usw. exakt in der betreffenden Kieferstellung visualisiert werden. Hierdurch können noch weitere Aussagen über eine mögliche Pathologie getroffen werden, die dem virtuellen Kieferabbild, welches auf den Kondylographiedaten basiert, selbst nicht oder nicht ausreichend entnehmbar sind. Der Vorteil liegt dann insbesondere darin, dass dieses neue Abbild die Situation in der im Kondylogramm für auffällig befundenen Situationen vollständig darstellen kann. Durch die erzwungene Relation der Zähne zueinander während des tomographischen Aufnahmeverfahrens sind besonders gut die Kiefergelenke mit dem Diskus und den beteiligten Weichgeweben anhand der volumentomographischen Aufnahme diagnostizierbar.

Um einen Diagnoseverdacht aus einer Kondylographie gezielt in einem bildgebenden System überprüfen zu können, kann mit dem vorliegenden Verfahren nun die Lagebeziehung der Kiefer zueinander, also einer bestimmten Kieferstellung, gezielt eingestellt werden. Wurde in den Kondylographiedaten zuvor eine Auffälligkeit zu einem bestimmten Zeitpunkt des Kaufvorganges festgestellt, so wird genau diese Lagebeziehung von Ober- und Unterkiefer, die zu diesem Zeitpunkt der Kieferbewegung gegeben war, auch in dem nunmehr erzeugten Abbild des Oberkiefers und des Unterkiefers vorherrschen. So kann eine sehr hohe Aussagekraft in der Kombination der Achsiographie mit einem bildgebenden Verfahren sichergestellt werden. Auch die Lagebeziehungen kurz vor bzw. kurz nach diesem bestimmten Zeitpunkt sind von großem Interesse in der Differenzialdiagnostik mit bildgebenden Verfahren.

Die oben beschriebene Fusionierung von Bewegungs- und Volumendaten ermöglicht nun auch die Aufbereitung der Kondylographiedaten anhand von anatomischen Verhältnissen. Beispielsweise kann die aus der Achsiographie bekannte Darstellung einer Achsspur nun auch anhand der anatomischen Verhältnisse parametrisiert werden. Anstatt die Achse und ihre Bewegungsspur rein aus der Kinematik der Bewegungsaufzeichnung herzuleiten, kann beispielsweise die - nur in der Tomographie sichtbare - Form der Kiefergelenkköpfchen sowie die Steilheit der Gelenkpfanne die Wahl der Achse und damit auch die Achsspur beeinflussen.

Es kann von jedem Zeitintervall der Bewegungsaufzeichnung nun die entsprechende Bewegung in den volumetrischen Tomographiedaten - z.B. durch Abspielen einer Animation der bewegten volumetrischen Tomographiedaten - dargestellt werden.

Vorteilhaft an der Fusionierung von Bewegungs- und Tomographiedaten ist auch, dass nun die Bewegungsbahn jedes Punktes des Unterkiefers nachvollzogen werden kann. Durch diese Information sind Darstellungen möglich, die dem Benutzer die Bewegungstrajektorie von einzelnen Punkten aufbereiten. Insbesondere kann die Darstellung so erfolgen, dass gar keine Animation abgespielt werden muss, sondern die Trajektorie von Punkten in der Zeit wiederum als Spur im Raum angezeigt wird. Auch die Auswahl und/oder bevorzugte Darstellung von anatomischen Punkten, die bestimmte kinematische Eigenschaften aufweisen ist so möglich. Beispielsweise können z.B. als ausgewähltes Element Punkte im Unterkiefer hervorgehoben werden, die sich i) besonders schnell bewegen, die sich ii) gleichförmig bewegen, die iii) besonders stark beschleunigt werden, die iv) eine besonders große oder besonders kleine Strecke zurücklegen oder sich gar nicht bewegen. Auch können Punkte auf dem Knochen und/oder auf den Zähnen ermittelt werden, die sich auf der linken und rechten Kieferseite bzgl. der oben beschriebenen kinematischen Kenngrößen symmetrisch bzw. asymmetrisch verhalten ("kinematische/funktionelle Symmetrie", in Unterscheidung zur morphologischen Symmetrie).

Umgekehrt kann die Tomographie nun verwendet werden, um interessante Bereiche in der Kondolygraphie (die ja meist aus mehreren Aufnahmeserien besteht) auszuwählen. Beispielsweise kann der Behandler auf einen Zahn oder auf einen Punkt als auszuwählendes Element am Zahn, Kieferknochen oder Kiefergelenk klicken und sich die Zeitbereiche und Spuren der Animation anzeigen lassen, in denen der angewählte Punkt eine der oben aufgezählten kinematischen Kenngrößen über- oder unterschreitet. So könnte z.B. für einen Punkt auf einem unteren Schneidezahn die Kondylographieaufnahme ausgewählt werden, in der dieser Schneidezahnpunkt am stärksten beschleunigt wird oder die Kondylographieaufnahme ausgewählt werden, in der dieser Zahnpunkt die größte Strecke zurücklegt.

Durch die beschriebene Fusionierung von digitalen Abbildern und Bewegungsdaten können die Bewegungsdaten erfindungsgemäß nun auch in allen Visualisierungen verwendet werden, die von volumetrischen Tomographiedaten abgeleitet sind. So können beispielsweise Projektionsaufnahmen aus den tomographischen Volumendaten berechnet werden, die kephalometrischen Röntgenaufnahmen in unterschiedlichen Kieferstellungen entsprechen, ohne den Patienten weiterer ionisierender Strahlung aussetzen zu müssen. Solche Röntgenaufnahmen können erfindungsgemäß auch erstmalig als patientenindividuelle Animation berechnet und dargestellt werden. Weiterhin kann die Durchstrahlrichtung solcher errechneter Röntgenaufnahmen nun abhängig von der aufgenommenen Bewegungsaufzeichnung gewählt werden. Besonders vorteilhaft ist beispielsweise eine Durchstrahlrichtung, die jeweils entlang der aus den Bewegungsdaten berechneten Scharnierachsrichtung der Kiefergelenke läuft; damit kann sich die Durchstrahlrichtung auch für jeden Zeitpunkt der Bewegungsaufzeichnung ändern.

Auch andere simulierte Aufnahmen können nun von der Bewegungsaufzeichnung profitieren. Beispielsweise kann nun auch eine Panoramaaufnahme oder eine Animation aus Panoramaaufnahmen aus den volumetrischen Volumendaten berechnet werden, die anhand der Bewegungsaufzeichnung parametrisiert wird. Beispielsweise könnte die Panoramaaufnahme so berechnet und angezeigt werden, dass sie möglichst symmetrisch bzgl. auf linker und rechter Seite des Kiefers gemessener Bewegungen ist. Ferner können auch Zahnfilmaufnahmen und Zahnfilmanimationen aus den volumetrischen Tomographiedaten berechnet werden, die beispielsweise die Zähne des Ober- und Unterkiefers zeigen wenn sie i) keinen Kontakt haben ii) einen Erstkontakt haben oder iii) in maximaler Interkuspidation sind. Ferner können in so berechneten Projektionsaufnahmen auch wiederum Bewegungsspuren von beliebigen Punkten der Zähne und Kiefer angezeigt werden, z.B. indem man mit einem Eingabegerät, insbesondere mit einer Maus - über einen Punkt in einer Projektionsaufnahme fährt.

Die Erfindung betrifft ferner eine Scanschiene, die durch ein Verfahren der oben genannten Art hergestellt ist.

Die Erfindung wird anhand der Figuren nachfolgend näher erläutert. Hierin zeigt
- Figuren 1a und 1b: eine Kondylographie von Ober- und Unterkiefer und die daraus berechnete Achsspur;
- Figur 2: ein Ablaufschema in einem Blockdiagramm;
- Figuren 3a - 3c: eine Fusionierung von Ober- und Unterkieferbewegungen und Tomographiedaten

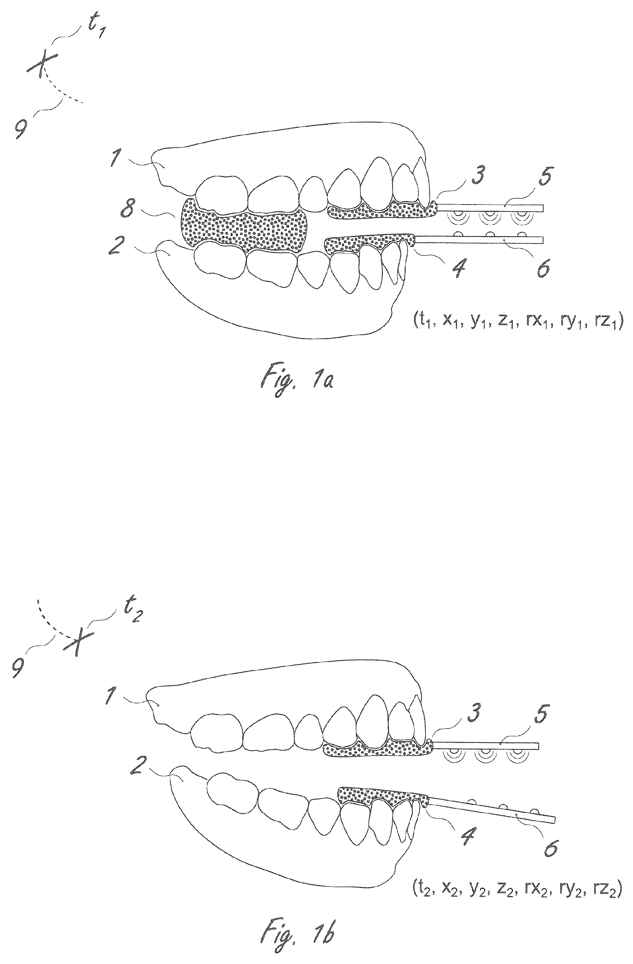

In Figur 1 ist die Kondylographie anhand zweier unterschiedlicher Kieferrelationen zu den Zeitpunkten t₁ bzw. t₂ dargestellt. Der Oberkiefer 1 ist dabei mit einem über ein oberes Gebissstück 3 starr verbundenen Aktorelement 5 versehen. Der Unterkiefer 2 ist mit einem über ein unteres Gebissstück 4 starr verbundenen Sensorelement 6 versehen. Natürlich können Aktorelement und Sensorelement am jeweils anderen Kiefer angebracht werden. Durch die starr auf Aktor- und Sensorelemente übertragene Kieferbewegungen und durch die Kenntnis der Aktor- und Sensorelementgeometrie kann anhand der im Sensorelement 6 gemessenen Signale auf die Relativbewegung von Ober- und Unterkiefer geschlossen werden. Für jeden Zeitpunkt tᵢ während der Kondylographie kann somit ein 6-Tupel gemessen werden, der die Translation und Rotation jedes Punktes im Raum dieser starren Transformation enthält. Aus diesen Daten kann für eine Bewegung oder für einen Bewegungsabschnitt eine gedachte Achsspur 9 berechnet werden. Durch ein Bissregistrat 8 kann die Kieferrelation zum Zeitpunkt t₁ in einen definitiven geometrischen Bezug gesetzt werden und dadurch zu jedem anderen Zeitpunkt tᵢ anhand der Kondylographie umgerechnet werden. Die Gebissstücke 3 und 4 sind bei einigen Systemen und Aufnahmeprotokollen auch so gestaltet, dass sie die Okklusalflächen der Zähne nicht bedecken. In so einem Falle kann die Bewegung der Zähne bis in die Schlussbissstellung (Interkuspidation) aufgezeichnet werden.

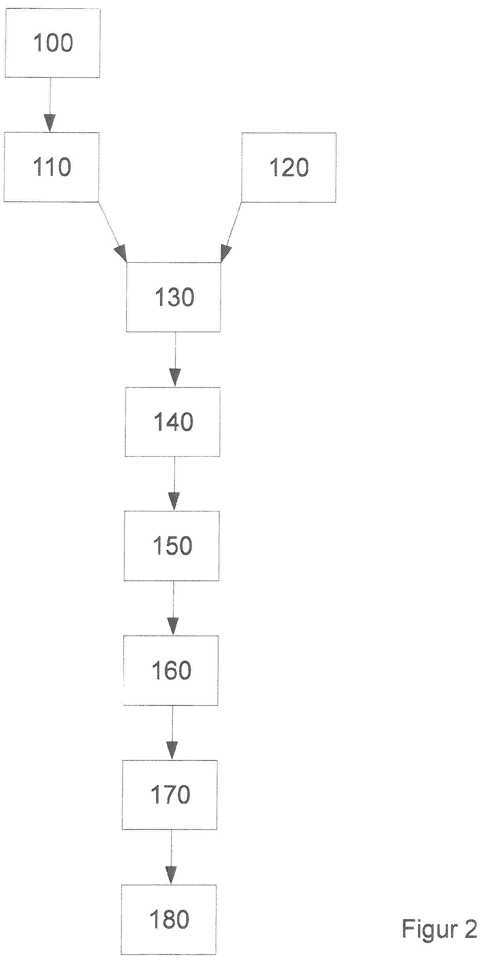

In Figur 2 wird nun das erfindungsgemäße Verfahren in einem Blockschaltbild schematisch erläutert. In einem ersten Schritt 100 wird ein Kondylographieverfahren durchgeführt, welches aus dem Stand der Technik bereits bekannt ist und in Figur 1 dem Grunde nach erläutert wurde. Somit wird also eine Vielzahl von Positionsdatensätzen aufgenommen, für die weitere Verwendung. Im Schritt 110 werden nun zu einem bestimmten Zeitpunkt des Kondylographieverfahrens Oberflächenabschnitte des realen Oberkiefers und des realen Unterkiefers aufgenommen, worin in diesem Abtastverfahren auch die relative Ausrichtung zwischen Unterkiefer und Oberkiefer zueinander berücksichtigt wird. Dies kann beispielsweise durch das Bissregistrat 8, wie in Figur 1 a gezeigt, erfolgen. Dabei beisst die Testperson auf das Bissregistrat, beispielsweise ein weiches Wachsmaterial, auf, beispielsweise zum Zeitpunkt t₁ in Figur 1, bei dem die Zähne z.B. fast geschlossen sind. Das Bissregistrat tastet dabei dann Oberflächenbereiche des Oberkiefers als auch des Unterkiefers ab in einer realen Situation während des Kauvorganges. Dieser Vorgang ist in dem Blockdiagramm nach Figur 2 mit dem Schritt 110 dokumentiert. Dabei wird aber auch festgehalten, dass genau zu diesem Zeitpunkt t₁ diese Abtastung erfolgt, sodass der Abdruck, der auf dem Bissregistrat 8 verbleibt, im unmittelbaren Zusammenhang zu dem Kondylogramm nach Figur 1 gebracht werden kann. Das Bissregistrat 8 wird dann eingescannt und digitalisiert.

Im Schritt 130 erfolgt nun die Fusionierung des Kondylogramms mit beliebigen digitalen volumetrischen Abbildern des Oberkiefers und des Unterkiefers. Dazu wurden zuvor im Schritt 120 digitale Oberkieferabbilder und digitale Unterkieferabbilder beispielsweise mittels eines Tomographieverfahrens aufgenommen. Allein aus der räumlichen Trennung des Astes mit den Funktionen 100 und 110 und dem Ast mit der Funktion 120 lässt sich bereits schließen, dass das Aufnehmen des Kondylogramms vollkommen unabhängig zur Aufnahme der digitalen volumetrischen Ober- und Unterkieferabbilder nach Verfahrensschritt 120 ist. Der Vorteil liegt darin, dass digitale volumetrischen Oberkiefer und Unterkieferabbilder verwendet werden können, die irgendwann einmal gemacht wurden. Für das Fusionieren der digitalen volumetrischen Ober- und Unterkieferabbilder, beispielsweise den Tomographieaufnahmen, mit den Kondylograhiedaten werden nun die abgetasteten Oberflächenbereiche des Bissregistrats 8 oder eines sonstigen Abtastverfahrens verglichen mit Oberflächendaten der digitalen volumetrischen Ober- und Unterkieferabbilder. Dabei werden die digitalen volumetrischen Ober- und Unterkieferbilder derart zueinander ausgerichtet, dass diese in Übereinstimmung mit den Oberflächenabschnitten des eingescannten Bissregistrats gelangen. Damit dies erfolgen kann, kann gegebenenfalls eine sogenannte Segmentierung in den volumetrischen Daten durchgeführt werden, die definiert, welche Teile der volumetrischen Daten dem beweglichen Unterkiefer entsprechen und welche nicht. Damit erfolgt dann eine Ausrichtung des digitalen volumetrischen Oberkieferabbildes und des digitalen volumetrischen Unterkieferabbildes zueinander derart, dass die Stellung der beiden Abbilder exakt derart zueinander ausgerichtet sind, wie es die realen Oberkiefer und Unterkiefer zu dem Zeitpunkt t₁ waren, als das Bissregistrat während des Kondylographieverfahrens erzeugt wurde.

Im Schritt 140 wird nun ein Positionsdatensatz für eine nähere Untersuchung ausgewählt, beispielsweise der Positionsdatensatz zum Zeitpunkt t₂ nahe der geöffneten Kieferstellung. Durch Verlagerung des digitalen volumetrischen Unterkieferabbildes gegenüber dem digitalen volumetrischen Oberkieferabbild im Schritt 150 wird damit das virtuelle Kieferabbild geschaffen, bei dem das digitale volumetrische Unterkieferabbild in einer gewünschten Stellung gegenüber dem digitalen volumetrischen Oberkieferabbild dargestellt ist. Diese Stellung entspricht einer realen Stellung, die tatsächlich während des Kondylographieverfahrens auch vom realen Kiefer eingenommen wurde.

An diesem ausgerichteten virtuellen Kieferabbild können nun weitere Untersuchungen oder Maßnahmen getroffen werden. Beispielsweise kann in diesem virtuellen Kieferabbild nun die Anordnung von Gelenkselementen im Kiefergelenk genau analysiert werden und auf mögliche Pathologien hin untersucht werden. Für diese Untersuchung können im Wesentlichen ausschließlich die Bildinformationen herangezogen werden, die durch die digitalen volumetrischen Ober- und Unterkieferabbilder vorhanden sind. Im Falle der Tomographie sind dies die knöchernen Strukturen, da sich diese einer starren Transformation folgend bewegen. Die Position der an der Bewegung beteiligten Weichteile kann jedoch nicht immer unmittelbar erschlossen werden. Sollte diese Untersuchung also nicht ausreichen, so können mit Hilfe dieses virtuellen Kieferabbildes weitere Schritte eingeleitet werden.

Insbesondere kann dieses virtuelle Kieferabbild zur Herstellung einer Scanschiene verwendet werden. Eine solche Scanschiene ist eine real existierende Schiene, die zwischen dem realen Oberkiefer und Unterkiefer platziert werden kann und damit den realen Oberkiefer in eine definierte Ausrichtung zum realen Unterkiefer bringen bzw. diesen in der definierten Ausrichtung halten kann. Dafür können Oberflächendaten aus dem virtuellen Kieferabbild extrahiert werden und daraus eine Oberfläche für die Scanschiene zunächst virtuell erzeugt werden. In dieser virtuellen Scanschiene werden damit also Anlageflächen für die Zähne oder sonstige Teile des Kiefers erzeugt, wobei die gewünschte relative Ausrichtung der Kiefer zueinander natürlich berücksichtigt bleibt. Durch ein Fräs-, Rapidprototype- oder ähnliches Verfahren zur Herstellung von realen Objekten kann dann diese virtuell hergestellte Scanschiene auch real umgesetzt werden. Setzt der Patient diese Scanschiene dann ein, so nimmt der Patient dann real die Kieferstellung ein, die der Kieferstellung im virtuellen Kieferabbild entspricht. Anhand dieser realen Kieferstellung können nun weitere Untersuchungen durchgeführt werden. Insbesondere kann dabei ein weiteres bildgebendes Verfahren zur Herstellung eines neuen volumetrischen Abbildes durchgeführt werden, beispielsweise ein Tomographieverfahren, in dem die Gelenke oder andere zu untersuchende Teile des Kiefers des Patienten in dieser konkret zu untersuchenden Stellung aufgenommen werden. In einem solchen Verfahren können dann auch (oder nur) Elemente aufgenommen werden, beispielsweise Muskeln, Knorpel, Sehnen, die flexibel sind und sich durch die vorherige Simulation eben nicht exakt nachstellen lassen. Das virtuelle Erzeugen der Scanschiene ist in Schritt 160 dargestellt. Das reale Erzeugen der realen Scanschiene wird in dem Schritt 170 visualisiert. Im Schritt 180 wird dann das Tomographieverfahren durchgeführt, bei dem der Patient die Scanschiene trägt.

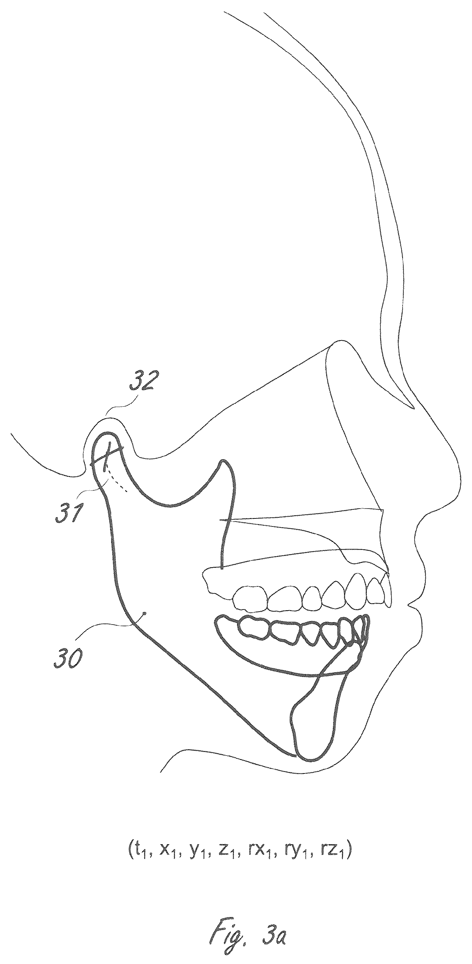

In der Figur 3a wird die Fusionierung von Kondylographiedaten und der digitalen volumetrischen Ober- und Unterkieferabbilder zum Zeitpunkt t₁ gezeigt. Die dicken Linien fassen die knöchernen anatomischen Bereiche des Unterkiefers zusammen, die sich gemeinsam als starres Objekt 30 während eines Kauvorganges bewegen. Durch diese starre Verbindung können nun die an den Zähnen gemessenen Bewegungen der Kondylographie auch auf die knöchernen Bereiche der in der Tomographie sichtbaren Anatomie übertragen werden. Insbesondere kann nun auch ein neuer Achspunkt samt Spur 31 zur Darstellung gewählt werden, der diese Anatomie berücksichtigt. Insbesondere kann die Wahl dieses "anatomischen" Achspunktes auch von der Geometrie der Gelenkgrube 32 oder von anderen anatomischen Strukturen abhängig gewählt werden.

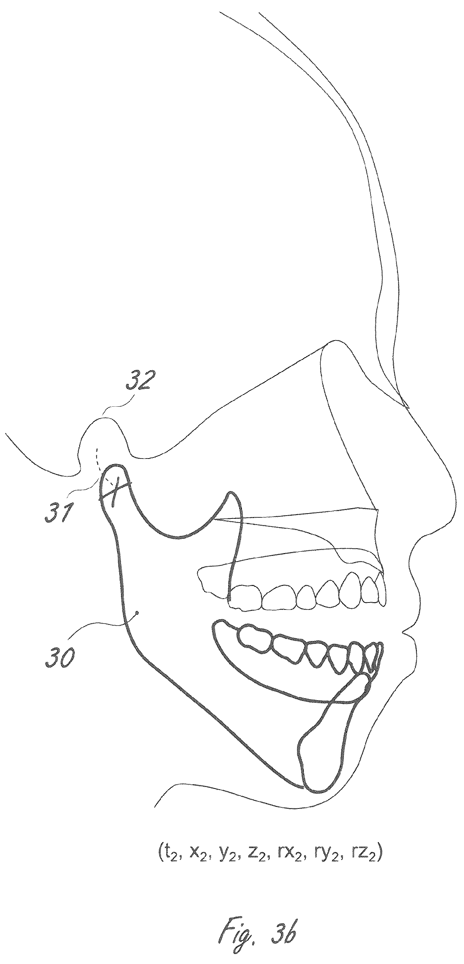

In der Figur 3b wird die Situation zum Zeitpunkt t₂ gezeigt. Durch die starre Verbindung von Zähnen und Knochen im Unterkiefer kann nun die durch die Kondylographie gemessene Bewegung auch auf die in der Tomographie dargestellte Knochenanatomie 30 übertragen werden. Damit können die knöchernen Teile des Unterkiefers 30 des zum Zeitpunkt t₁ erzeugten Tomogramms so verschoben werden, als sei das Tomogramm in der Kieferrelation des Zeitpunktes t₂ aufgenommen worden.

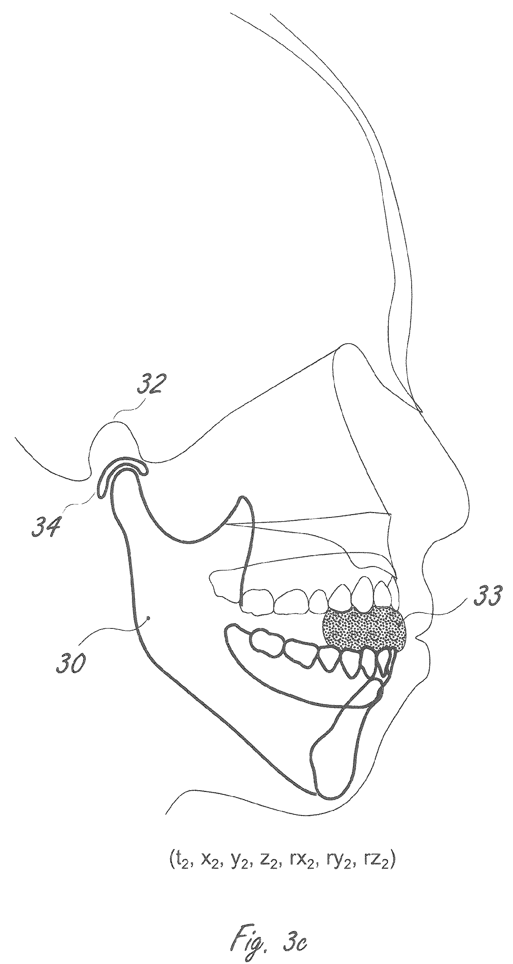

In der Figur 3c wird exemplarisch ein Weichgewebsteile 34 (Diskus) gezeigt, dessen Position und Form nicht allein durch die Anwendung einer starren Transformation ermittelt werden kann. Durch den Einsatz der erfindungsgemäßen Scanschiene 33 kann nun aber ein (weiteres) Tomogramm erzeugt werden, das die genaue Position und Form des Weichgewebsteils 34 abbilden kann.

## Patentansprüche

1. Verfahren zum Erstellen eines virtuellen volumetrischen Kieferabbildes, welches die Anordnung von Gelenkselementen im Kiefergelenk umfasst, , wobei ein digitales, volumetrisches Unterkieferabbild in unterschiedlichen Stellungen gegenüber einem digitalen, volumetrischen Oberkieferabbild dargestellt werden kann, umfassend die folgenden Verfahrensschritte:
Durchführen (100) eines Bewegungsaufzeichnungsverfahrens, insbesondere ein Kondylographieverfahren, zum Erhalt einer Bewegungsaufzeichnung, insbesondere eines Kondylogramms, worin eine Vielzahl von Positionsdatensätzen ((t₁, x₁, y₁, z₁, rx₁, ry₁, rz₁), (t₂, x₂, y₂, z₂, rx₂, ry₂, rz₂) , ...) über einen definierten Zeitraum erzeugt werden, wobei ein Positionsdatensatz eine reale räumliche Stellung eines Unterkiefers gegenüber einem Oberkiefer zu einem bestimmten Zeitpunkt beschreibt;
Abtasten (110) von Oberflächenabschnitten des realen Oberkiefers und des realen Unterkiefers zu einem definierten Zeitpunkt während des Bewegungsaufzeichnungsverfahrens, insbesondere mittels eines Bissregistrats, zum Erhalt einer räumlichen Relation der Oberflächenabschnitte während des definierten Zeitpunkts;
Zuordnen (130) zumindest eines Positionsdatensatzes einer virtuellen Stellung des digitalen Unterkieferabbildes gegenüber dem digitalen Oberkieferabbild, insbesondere mittels des Bissregistrats, welches digitalisiert wurde;
Auswählen (140) eines Positionsdatensatzes, und
virtuelles Ausrichten (150) des digitalen Unterkieferabbildes gegenüber dem digitalen Oberkieferabbild entsprechend des ausgewählten Positionsdatensatzes, wodurch das virtuelle Kieferabbild erzeugt wird, wobei die virtuelle Ausrichtung einer realen Stellung des Unterkiefers gegenüber dem Oberkiefer entsprechend des ausgewählten Positionsdatensatzes entspricht so dass das Kiefergelenks in einer zu begutachtenden, realen Stellung angezeigt wird.

2. Verfahren nach dem vorherigen Anspruch,
**dadurch gekennzeichnet,**
**dass** das digitale Oberkieferabbild und das digitale Unterkieferabbild während einer beliebigen Kieferstellung jeweils durch ein Bildgebungsverfahren erzeugt werden (120).

3. Verfahren nach einem der vorherigen Ansprüche,
**dadurch gekennzeichnet,**
**dass** zum Auswählen des Positionsdatensatzes ein Zeitpunkt in der Bewegungsaufzeichnung ausgewählt wird, und der ausgewählte Positionsdatensatz anhand des ausgewählten Zeitpunktes identifiziert wird.

4. Verfahren nach einem der vorherigen Ansprüche,
**dadurch gekennzeichnet,**
**dass** ein Element des digitalen Ober- oder Unterkieferabbildes definiert ausgewählt wird, und dass eine Bewegungsspur des ausgewählten Elements entsprechend der Bewegungsaufzeichnung angezeigt wird.

5. Verfahren nach einem der vorherigen Ansprüche,
**dadurch gekennzeichnet,**
**dass** die Bewegungspur von Elementen des digitalen Ober- oder Unterkieferabbildes während eines Bewegungsablaufs entsprechend der Bewegungsaufzeichnung statistisch ausgewertet wird.

6. Verfahren zum Erzeugen einer Scanschiene, wobei zunächst ein Verfahren zum Erstellen eines virtuellen Kieferabbildes nach einem der vorherigen Ansprüche durchgeführt wird,
**dadurch gekennzeichnet,**
**dass** ferner anhand des virtuellen Kieferabbildes eine Scanschiene erzeugt wird, wobei die Scanschiene zumindest eine definierte Oberkieferanlagefläche und eine definierte Unterkieferanlagefläche aufweist, wobei die Oberkieferanlagefläche und die Unterkieferanlagefläche derart zueinander ausgerichtet sind, dass der Unterkiefer zum Oberkiefer die räumliche Stellung entsprechend des ausgewählten Positionsdatensatzes einnimmt, wenn die Oberkieferanlagefläche in Anlage mit dem Oberkiefer und die Unterkieferanlagefläche in Anlage mit dem Unterkiefer ist.

7. Verfahren zum Erzeugen eines digitalen Abbildes, **dadurch gekennzeichnet, dass** eine Scanschiene, die nach dem vorherigen Anspruch hergestellt wurde, in Anlage mit dem realen Unterkiefer und dem realen Oberkiefer gebracht wird und anschließend der reale Oberkiefer und der reale Unterkiefer mit jeweils angelegter Scanschiene einem, insbesondere tomographischen, Bildaufnahmeverfahren zugeführt werden, und dass durch das Bildaufnahmeverfahren ein Abbild erzeugt wird, bei dem Körperteile insbesondere des Kieferbereichs entsprechend des ausgewählten Positionsdatensatzes dargestellt sind.

## Claims

1. Method of producing a virtual volumetric jaw image which comprises the arrangement of joint elements in the mandibular joint, wherein a digital, volumetric lower jaw image can be shown in different positions with regard to a digital volumetric upper jaw image, comprising the follow procedures:
implementation (100) of a movement recording process, more particularly a condylography procedure in order to obtain a movement recording, more particularly a condylogram, wherein a plurality of position data sets ((t_{1,} x₁, y₁, z₁, rx₁, ry₁, rz₁), ((t₂, x₂, y₂, z₂, rx₂, ry₂, rz₂),...) are created over a defined period of time, wherein a position data set describes an actual spatial position of a lower jaw in relation to an upper jaw at a particular point in time;
scanning (110) of surface sections of the actual upper jaw and the actual lower jaw at a defined point in time during the movement recording procedure, more particularly by means of bite registration, in order to obtain a spatial relationship of the surface sections during the defined point in time;
allocation (130) of at least one position data set to a virtual position of the digital lower jaw image in relation to the digital upper jaw image, more particularly by means of the digitalised bite registration;
selection (140) of a position data set and
virtual alignment (150) of the digital lower jaw image with regard to the digital upper jaw image in accordance with the selected position data set, as a result of which the virtual jaw image is created, wherein the virtual alignment corresponds to an actual position of the lower jaw in relation the upper jaw in accordance with the selected position data set so that the mandibular joint is shown in an assessable actual position.

2. Method according to the previous claim
**characterised in that**
the digital upper jaw image and the digital lower jaw image are each created by means of an imaging method during any position of the jaw (120).

3. Method according to any one of the previous claims
**characterised in that**
in order to select the position data set a point in time during the movement recording is selected and the selected position data set is identified by way of the selected point in time.

4. Method according to any one of the previous claims
**characterised in that**
one element of the digital upper or lower law images is selected in a defined manner and that a movement trace of the selected element corresponding to the movement recording is displayed.

5. Method according to any one of the preceding claims
**characterised in that**
the movement trace of elements of the digital upper or lower jaw image is statistically evaluated during the course of a movement in accordance with the movement recording.

6. Method of producing a scanning splint, wherein initially a method of producing a virtual jaw image in accordance with the preceding claims is implemented,
**characterised in that**
by means of the virtual jaw image a scanning splint is Iso produced, wherein the scanning splint has at least one defined upper jaw contact surface and one defined lower jaw contact surface, wherein the upper jaw contact surface and the lower jaw contact surface are aligned with regard to each other in such a way that with regard to the upper jaw the lower jaw assumes the spatial position in accordance with the selected position data set when the upper jaw contact surface is in contact with the upper jaw and the lower jaw contact surface is in contact with the lower jaw.

7. Method of creating a digital image **characterised in that** a scanning splint, which has been produced in accordance with the preceding claim, is brought into contact with the actual lower jaw and the actual upper jaw, after which the actual upper jaw and the actual lower jaw, both with the scanning splint in place, undergo an imaging procedure, more particularly a tomographic imaging procedure, and that by way of the imaging procedure an image is produced in which body parts, more particularly of the mandibular area, are shown in accordance with the selected position data set.

## Revendications

1. Procédé d'établissement d'une représentation volumétrique virtuelle des mâchoires, qui comprend la disposition d'éléments d'articulation dans l'articulation de la mâchoire, dans lequel une représentation volumétrique numérique de la mâchoire inférieure par rapport à une représentation volumétrique numérique de la mâchoire supérieure peut être représentée dans différentes positions, comprenant les étapes opératoires suivantes :
réalisation (100) d'un procédé d'enregistrement de mouvement, en particulier un procédé de condylographie, pour obtenir un enregistrement de mouvement, en particulier un condolygramme, une multitube d'ensembles de données de position ((t₁, x₁, y₁, z₁, rx₁, ry₁, rz₁), (t₂, x₂, y₂, z₂, rx₂, ry₂, rz₂), ...) étant générées pendant une période définie, un ensemble de données de position décrivant une position spatiale réelle d'une mâchoire intérieure par rapport à une mâchoire supérieure à un certain moment ;
balayage (110) de sections superficielles de la mâchoire supérieure réelle et de la mâchoire inférieure réelle à un moment défini pendant le procédé d'enregistrement de mouvement, en particulier au moyen d'un enregistrement d'occlusion, pour obtenir une relation spatiale entre les sections superficielles pendant le moment défini ;
association (130) d'au moins un ensemble de données de position d'une position virtuelle de la représentation numérique de la mâchoire inférieure par rapport à la représentation numérique de la mâchoire supérieure, en particulier au moyen de l'enregistrement d'occlusion qui a été numérisé ;
sélection (140) d'un ensemble de données de position, et
alignement virtuel (150) de la représentation numérique de la mâchoire inférieure par rapport à la représentation numérique de la mâchoire supérieure en fonction de l'ensemble de données de position sélectionné, ce qui a pour effet de créer la représentation virtuelle des mâchoires, l'orientation virtuelle d'une position réelle de la mâchoire inférieure par rapport à la mâchoire supérieure correspondant à l'ensemble de données de position sélectionné, de sorte que l'articulation de la mâchoire est affichée dans une position réelle à examiner.

2. Procédé selon la revendication précédente,
**caractérisé en ce que**
la représentation numérique de la mâchoire supérieure et la représentation numérique de la mâchoire inférieure sont créées dans n'importe quelle position des mâchoires respectivement par un procédé d'imagerie (120).

3. Procédé selon une des revendications précédentes,
**caractérisé en ce que**,
pour sélectionner l'ensemble de données de position, un moment de l'enregistrement de mouvement est sélectionné et l'ensemble de données de position est identifié à partir du moment sélectionné.

4. Procédé selon une des revendications précédentes,
**caractérisé en ce**
**qu'**un élément de la représentation numérique de la mâchoire supérieure ou inférieure est sélectionné de manière définie et qu'une piste de mouvement de l'élément sélectionné est affichée en fonction de l'enregistrement du mouvement.

5. Procédé selon une des revendications précédentes,
**caractérisé en ce que**
la piste de mouvement des éléments de la représentation numérique de la mâchoire supérieure ou inférieure est exploitée statistiquement pendant un cycle de mouvement en fonction de l'enregistrement du mouvement.

6. Procédé de création d'un rail de balayage, dans lequel un procédé d'établissement d'une représentation virtuelle des mâchoires selon une des revendications précédentes est d'abord réalisé,
**caractérisé en ce que**
en partant de la représentation virtuelle des mâchoires, un rail de balayage est en outre créé, le rail de balayage présentant au moins une surface de contact de mâchoire supérieure définie et une surface de contact de mâchoire inférieure définie, la surface de contact de mâchoire supérieure et la surface de contact de mâchoire inférieure étant orientées l'une par rapport à de manière à ce que la mâchoire inférieure prenne, par rapport à la mâchoire supérieure, la position spatiale correspondant à l'ensemble de données de position sélectionné lorsque la surface de contact de la mâchoire supérieure est en contact avec la mâchoire supérieure et la surface de contact de la mâchoire inférieure est en contact avec la mâchoire inférieure.

7. Procédé de création d'une représentation numérique, **caractérisé en ce qu'**un rail de balayage qui a été réalisé selon la revendication précédente est amené en contact avec la mâchoire inférieure réelle et la mâchoire supérieure réelle puis que la mâchoire supérieure réelle et la mâchoire inférieure réelle sont amenées avec le rail de balayage respectivement posé dessus vers un procédé de prise d'images, en particulier tomographique, et qu'une image est prise par le procédé de prise d'images, image dans laquelle des parties du corps, en particulier de la zone des mâchoires, sont représentées en fonction de l'ensemble de données de position sélectionné.
